(19) Europäisches Patentamt
European Patent Office
Office européen des brevets

(11) Publication number: 0 004 107
B1

(12) **EUROPEAN PATENT SPECIFICATION**

(45) Date of publication of patent specification: 07.04.82

(51) Int. Cl.³: C 07 D 209/52,
A 01 N 43/38

(21) Application number: 79200096.0

(22) Date of filing: 27.02.79

# (54) 3-Azabicyclo(3.1.0)hexane derivatives, a process for their preparation, biologically active compositions containing them and herbicidal method using them.

(30) Priority: 01.03.78 GB 806678
20.04.78 GB 1567778

(43) Date of publication of application:
19.09.79 Bulletin 79/19

(45) Publication of the grant of the patent:
07.04.82 Bulletin 82/14

(84) Designated Contracting States:
BE CH DE FR GB IT NL SE

(56) References cited:
FR - A - 2 324 236

CHEMICAL ABSTRACTS, vol. 77, no. 1, July 3, 1972, Columbus, Ohio, USA
I. ROWLAND et al. "Inhibition of bacterial growth by cis-and trans-3,4-methano-L-proline", page 110, abstract 14817y

(73) Proprietor: SHELL INTERNATIONALE RESEARCH MAATSCHAPPIJ B.V.
Carel van Bylandtlaan 30
NL-2596 HR DEN HAAG (NL)

(72) Inventor: Mason, Ronald Frank
"Kingswood" Westwell
nr Ashford Kent (GB)
Inventor: Devlin, Barry Roy John
59 Ruins Barn Road
Sittingbourne Kent (GB)

(74) Representative: Rogers, Roy Charles et al.
4 York Road
London SE1 7NA (GB)

EP 0 004 107 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# 3-Azabicyclo(3.1.0)hexane derivatives, a process for their preparation, biologically active compositions containing them and herbicidal method using them

The invention relates to 3-azabicyclo[3.1.0]hexane derivatives, a process for their preparation, biologically active compositions containing them and herbicidal method using them. The compounds according to the invention exhibit herbicidal properties, and are useful intermediates in the preparation of 2-carboxy-3-azabicyclo[3.1.0]hexane and derivatives thereof.

FR—A—2324236 teaches that 2-carboxy-3-azabicyclo[3.1.0]hexane and certain derivatives thereof have plant growth regulating properties, being especially useful as chemical agents for sterilising male anthers in plants.

The present invention provides a compound of the general formula:

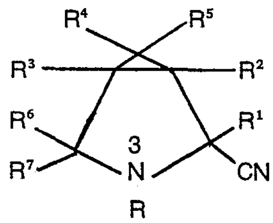

(I)

wherein R represents a hydrogen atom or an alkoxycarbonyl, alkyl or cycloalkyl group which may be unsubstituted or substituted by one or more of the same or different substituents selected from halogen atoms, alkoxy groups and aryl groups; $R^1$ represents a hydrogen atom or an alkyl or cycloalkyl group which may be unsubstituted or substituted by one or more of the same or different substituents selected from halogen atom and alkoxy groups; and each of $R^2$, $R^3$, $R^4$, $R^5$, $R^6$ and $R^7$ independently represents a hydrogen or halogen atom or an alkyl, cycloalkyl, alkenyl, aryl, alkaryl or aralkyl group which may be unsubstituted or substituted by one or more of the same or different substituents selected from halogen atoms and alkoxy groups, or each of $R^2$, $R^3$, $R^6$ and $R^7$ independently has one of these meanings and $R^4$ and $R^5$ together represent an alkylene group, or each of $R^2$, $R^3$, $R^4$ and $R^5$ independently has one of these meanings and $R^6$ and $R^7$ together represent an alkylene group; or an acid addition salt thereof.

Unless otherwise stated, any halogen atom present in a compound according to the invention is preferably a chlorine or fluorine atom. Any alkyl, alkenyl, or alkoxy group preferably has up to 6, especially up to 3, carbon atoms. Any aryl moiety is preferably a phenyl moiety, and any alkaryl or aralkyl group preferably has 7 or 8 carbon atoms.

R may for example represent a hydrogen atom or an alkyl group which may be unsubstituted or substituted by one or more of the same or different substituents selected from halogen atoms, alkoxy groups and aryl groups. If R represents an alkoxycarbonyl group, this group is preferably unsubstituted and preferably has up to 6 carbon atoms. R may for example represent an ethoxycarbonyl group. Preferably, R represents a hydrogen atom, an alkyl group having up to 6 carbon atoms which may be unsubstituted or substituted by up to 3 halogen atoms, an alkoxycarbonyl group having up to 6 carbon atoms, a cycloalkyl group, or a benzyl group which may be unsubstituted or substituted in the ring by up to 3 halogen atoms. More preferably, R represents a hydrogen atom or an alkyl group having up to 4 carbon atoms. Most preferably, R represents a methyl group or, especially, a hydrogen atom.

Preferably, $R^1$ represents a hydrogen atom or an alkyl group having up to 6 carbon atoms which may be unsubstituted or substituted by up to 3 halogen atoms. More preferably, $R^1$ represents a hydrogen atom or an alkyl group having up to 4 carbon atoms. Most preferably, $R^1$ represents a methyl group or, especially, a hydrogen atom.

Preferably, each of $R^2$, $R^3$, $R^4$, $R^5$, $R^6$ and $R^7$ independently represents a hydrogen or halogen atom, an alkyl or alkenyl group having up to 6 carbon atoms which may be unsubstituted or substituted by up to 3 halogen atoms, or an aryl, aralkyl or alkaryl group having up to 10 carbon atoms which may be unsubstituted or substituted by up to 3 halogen atoms, or each of $R^2$, $R^3$, $R^6$ and $R^7$ independently has one of these meanings and $R^4$ and $R^5$ together represent an alkylene group having up to 6 carbon atoms, or each of $R^2$, $R^3$, $R^4$, and $R^5$ independently has one of these meanings and $R^6$ and $R^7$ together represent an alkylene group having up to 6 carbon atoms. More preferably, each of $R^2$, $R^3$, $R^4$, $R^5$, $R^6$ and $R^7$ independently represents a hydrogen, fluorine, chlorine or bromine atom or an alkyl or alkenyl group having up to 4 carbon atoms which may be unsubstituted or substituted by up to 3 fluorine, chlorine or bromine atoms, for example a methyl, isobutenyl, difluorovinyl or dichlorovinyl group, or each of $R^2$, $R^3$, $R^6$ and $R^7$ independently has one of these meanings and $R^4$ and $R^5$ together represent an alkylene group having 4 or 5 carbon atoms, or each of $R^2$, $R^3$, $R^4$ and $R^5$ has one of these meanings and $R^6$ and $R^7$ together represent an alkylene group having 4 or 5 carbon atoms. Most preferably, each of $R^2$, $R^3$, $R^4$ and $R^5$ represents a hydrogen atom.

An especially preferred compound according to the invention is 2-cyano-3-azabicyclo[3.1.0]hexane.

The compounds of the general formula I form acid addition salts with organic and inorganic acids. Suitable salts include salts with mineral acids, for example hydrohalide, especially hydrochloride, salts. Acid addition salts should, of course, be prepared under conditions which do not hydrolyze the 2-cyano group.

The invention also provides a process for the preparation of a compound of the general formula I, characterized in that a compound of the general formula:

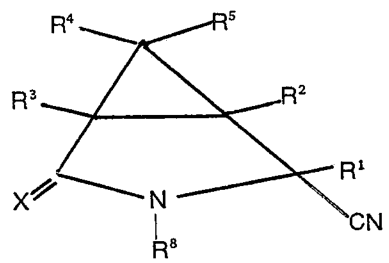

(II)

in which $R^1$, $R^2$, $R^3$, $R^4$ and $R^5$ have the meanings given for the general formula I, X represents an oxygen or sulphur atom, and $R^8$ represents a hydrogen atom or an alkyl, cycloalkyl, aryl, alkaryl or aralkyl group which may be unsubstituted or substituted by one or more of the same or different substituents selected from halogen atoms, alkoxy groups and aryl groups, is reacted with a trialkyl oxoniumfluoroborate, and at least part of the resulting product is reacted with a suitable reducing agent; and if desired, converting the resulting compound of the general formula I into another compound of the general formula I.

The reaction of the compound of the general formula II with a trialkyloxonium tetrafluoroborate produces a fluoroborate complex, which may be isolated, for example by crystallization, or used *in situ* for the subsequent reduction. Suitably the reaction is carried out at a temperature in the range of from 0 to 30°C, for example at room temperature. The reaction may conveniently be carried out in the presence of a suitable solvent, for example a liquid alkane or halogenated alkane, for example pentane, hexane, methylene chloride, chloroform or carbon tetrachloride. Mixtures of solvents may be suitable.

The trialkyloxonium tetrafluoroborate may, for example, be trimethyloxonium tetrafluoroborate, triethyloxonium tetrafluoroborate, or a mixed lower alkyl oxonium tetrafluoroborate.

The subsequent reduction step is carried out using a mild reducing agent, for example a simple or complex alkali metal hydride, for example sodium hydride, lithium hydride, lithium borohydride or, especially, sodium borohydride. Preferably, the reduction is carried out at ambient temperature. The reducing agent may be added directly to the reaction mixture obtained in the previous step, or it may be reacted with the tetrafluoroborate complex which has been isolated and dissolved or suspended in a suitable solvent, such as methanol or ethanol. The reaction mixture may be worked up by conventional procedures.

The product of the reduction is a compound of the general formula I in which each of $R^6$ and $R^7$ represents a hydrogen atom, and R represents a group other than an alkoxycarbonyl group. This resulting compound can be converted into a compound in which $R^6$ and/or $R^7$ represents a substituent other than a hydrogen atom by methods analogous to those known in the art, if necessary temporarily blocking other reactive sites in the molecule. Compounds in which R represents an optionally-substituted alkoxycarbonyl group can be prepared by reacting a resulting compound of formula I in which R represents a hydrogen atom with a halo-formate of the general formula:

$$Hal — CO_2 — R^9 \quad (V)$$

in which Hal represents a chlorine or bromine atom and $R^9$ represents an alkyl group which may be unsubstituted or substituted by one or more of the same or different substituents selected from halogen atoms, alkoxy groups and aryl groups. This reaction is suitably carried out at a temperature in the range of from −20°C to +20°C, in the presence of a suitable solvent, for example water. Suitably, the hydrogen halide produced during the reaction is neutralized using a base, for example potassium carbonate.

The compounds of the general formula II are described in European Patent Application No. 3866. They may be prepared by a process in which a compound of the general formula:

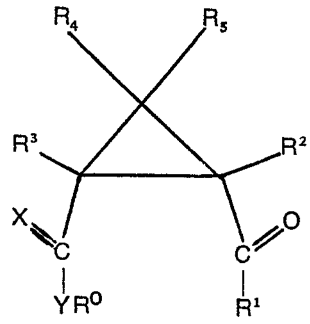

(III)

in which, $R^1$, $R^2$, $R^3$, $R^4$ and $R^5$ have the meanings given for the general formula I, each of X and Y independently represents an oxygen or sulphur atom, $R^0$ represents an optionally substituted alkyl group, an alkali metal ion, an ammonium ion, optionally substituted by one or more alkyl groups, or one equivalent of an alkaline earth metal ion, and the groups —$COR^1$ and —$CXYR^0$ are in cis-relationship to each other, is reacted with a cyanide in the presence of a compound of the general formula:

$$R^8NH_2 \quad (IV)$$

in which $R^8$ has the meaning given for the general formula II.

The compounds according to the invention exist in the form of geometric and optical isomers, the number of isomers depending on the meanings of the various substituents in the molecule. For example, the —CN group in the 2-position of the molecule may be cis or trans to the —$CR^4R^5$ group, and each of these isomers exists as a pair of optical isomers. The invention should be understood to include individual isomers and mixtures thereof.

If a mixture of isomers of the compound of formula II is used as starting material, the process according to the invention generally leads to a mixture of isomers of the compound of formula I, which may, if desired, be separated into individual isomers or groups of isomers by conventional methods. For example, cis and trans isomers may be separated by fractional crystallization or layer or column chromatography. However, depending on the isomer desired it may be advantageous to start from a particular isomer of a compound of the formula II. Especially preferred compounds according to the invention are those in which the —CN group is cis to the —$CR^4R^5$ group, for example cis (d,l) 2-cyano-3-azabicyclo[3.1.0]hexane itself, which may for example be obtained from cis (d,l) 2-cyano-3-azabicyclo[3.1.0]hexan-4-one.

Preparation of compounds of the general formula I in which R represents an optionally substituted alkoxycarbonyl group, provides a useful method of preparing single geometric isomers of compounds of the general formula I and derivatives thereof, since mixtures of isomers of these N-alkoxycarbonyl compounds can be separated into single isomers by fractional distillation. An especially useful process comprises reacting a mixture of the cis and trans isomers of 2-cyano-3-azabicyclo[3.1.0]hexane with a suitable ester of the general formula V, preferably ethyl chloroformate, and separating the resulting cis and trans isomers by fractional distillation. Hydrolysis of each of these isomers leads to the separate cis and trans isomers of 2-carboxy-3-azabicyclo[3.1.0]hexane.

The compounds of the general formula I are useful intermediate compounds in the preparation of 2-carboxy-3-azabicyclo[3.1.0]hexane and certain of its derivatives. These compounds, for example those described in German Offenlegungsschrift No. 2,641,295, exhibit interesting pollen suppressant and plant growth regulating activity. For example, 2-cyano-3-azabicyclo[3.1.0]hexane can be converted into the corresponding acid by hydrolysis, using for example a mineral acid, or into an ester, by reaction with an alcohol. Particular isomers of 2-carboxy-3-azabicyclo[3.1.0]hexane and its derivatives may be obtained. For example, trans (d,l) 2-cyano-3-azabicyclo[3.1.0]hexane can be converted into trans (d,l) 2-carboxy-3-azabicyclo[3.1.0]hexane by acid hydrolysis or into trans (d,l) 2-ethoxycarbonyl-3-azabicyclo[3.1.0]hexane by reaction with ethanol. The compounds according to the present invention can also be converted into the corresponding thioamides by reaction with hydrogen sulphide or a mono- or dialkyl sulphide in the presence of a tertiary amine.

The compounds of the general formula I exhibit herbicidal activity. The present invention, therefore, also provides a herbicidal composition comprising a carrier and/or surface-active agent, characterized in that it contains as active ingredient at least one compound of the general formula I. The invention also provides a method of controlling undesired plant growth at a locus, characterized in that there is applied to the locus a herbicidally effective amount of a compound or a composition according to the invention.

Any of the carrier materials or surface-active agents usually applied in formulating pesticides may be used in the compositions according to the present invention. Examples of such carriers and surface-active agents are given in British Patent Specification No. 1,293,546.

The following Examples illustrate the invention.

## Example 1

Preparation of 2-cyano-3-azabicyclo[3.1.0]hexan-4-one

A 250 ml flask was charged with 21.3 g cis 1-ethoxycarbonyl-2-formylcyclopropane (0.15 mol.), absolute ethanol (50 ml) and 4 drops of piperidine. The reaction contents were cooled to 0°C. Then 6 ml hydrogen cyanide (4.2 g; 0.155 mol.) were added and the reaction mixture was saturated with anhydrous ammonia and allowed to attain room temperature. After the addition of further ammonia the temperature rose slowly and the mixture was kept at 70—75°C for 45 minutes. After removal of the volatile components in a film evaporator under reduced pressure, the remaining mixture was saturated with ethanol while cooling in an ice-bath, then filtered and recrystallized from 30 ml ethanol.

5.2 g of product, m.p. 135—136°C were obtained. This product was characterized by proton and $C^{13}$ nuclear magnetic resonance spectroscopy as the pure cis compound.

Analysis:
Calculated for $C_6H_6N_2O$: C 59.0; H 5.0; N 22.9%
Found:- C 59.1; H 5.1; N 23.0%

From the mother liquor a further amount of 10.6 g 2-cyano-3-azabicyclo[3.1.0]hexan-4-one was obtained using chromatography over silica gel (methylene dichloride as eluent) as a cis/trans mixture. An analytical pure sample of the trans isomer (m.p. 89—90°C) was obtained using liquid-liquid chromatography. The compound was characterized by nuclear magnetic resonance spectroscopy. The total yield of products was 86%.

Similar results were obtained when the reaction was carried out using 0.675 mol. of cis 1-ethoxycarbonyl-2-formylcyclopropane as starting material (66% of cis/trans product isolated).

## Example 2

Preparation of cis-2-cyano-3-azabicyclo[3.1.0]hexane and the corresponding acid from cis 2-cyano-3-aza-bicyclo[3.1.0]hexan-4-one

Cis 2-cyano-3-azabicyclo[3.1.0]hexan-4-one prepared as in Example 1 (10 g, 0.08 mole) was dissolved in dry methylene dichloride (50 ml) and treated with triethyloxonium tetrafluoroborate (19 g, 0.1 mole) while stirring at 10—15°C. The stirring was continued for 18 hours during which time a crystalline solid separated. The reaction mixture was evaporated to dryness under reduced pressure and redissolved in dry ethanol (60 ml), cooled to between 5 and 10°C and treated portion-wise over 10 minutes with sodium borohydride (4.0 g, 0.105 mole). The mixture was then stirred for 20 hours at ambient temperature and then evaporated to dryness. Water (150 ml) was added and the solution obtained extracted several times with diethyl ether (total volume 250 ml). The solution was dried over magnesium sulphate and then evaporated to give 6.7 g of the crude nitrile.

This product was dissolved in 6 N HCl (80 ml) and refluxed for 4 hours. Having evaporated the solution down to a volume of about 20 ml, it was poured down a DOWEX 50 W—X8 (trade mark) column and washed until free of chloride ions. The product was eluted with 2N $NH_4OH$, collecting a total volume of 3 litres. After evaporation a syrupy residue remained which was heated with ethanol (100 ml) to deposit ammonium chloride. After evaporation of the solvent 2.5 g of a pale yellow oil were obtained which solidified under reduced pressure (1 mm). The N.M.R. and I.R. spectra of the product were identical to those of the natural product cis 3-azabicyclo[3.1.0]hexane-2-carboxylic acid. The cis content of the product was more than 90%.

In a similar experiment, starting from 4.5 g cis 2-cyano-3-azabicyclo[3.1.0]hexan-4-one a semisolid residue was obtained to which benzene was added. Evaporation of the benzene solution left a yellow oil which gave on distillation 1.2 g of cis 2-cyano-3-azabicyclo[3.1.0]hexane (b.p. 80—86°C at a pressure of 1 mm Hg).

## Example 3

Preparation of cis and trans 2-cyano-3-azabicyclo[3.1.0]hexane from a mixture of cis and trans 2-cyano-3-azabicyclo[3.1.0]hexan-4-one

A mixture of 45% cis and 55% trans 2-cyano-3-azabicyclo[3.1.0]hexan-4-one (18 g, 0.148 mole) was stirred in dry methylene dichloride (250 ml) and treated portion-wise with pure triethyloxonium tetrafluoroborate (32 g, 0.168 mole). Cooling was not required. After stirring for 30 minutes a heavy oil began to separate and the mixture was stirred at ambient temperature for 15 hours.

After evaporating the mixture to dryness under reduced pressure a yellow oil remained which was redissolved in dry ethanol (120 ml). The solution was stirred and cooled to 0°C and treated portion-wise with sodium borohydride (12.8 g, 0.336 mole). The mixture was stirred overnight, evaporated to a low volume and treated with water (100 ml). Repeated extractions of the solution with diethyl ether (total volume 600 ml) followed by evaporation of the extracts left a viscous yellow oil. The oil was redissolved in benzene, dried over magnesium sulphate and evaporated to dryness leaving a viscous oil which was distilled under reduced pressure and from which 7.1 g (44%) product (b.p. 56—60°C at a pressure of 0.3 mm Hg) was obtained.

Analysis:
Calculated for $C_6H_8N_2$: C 66.6; H 7.5; N 25.9%
Found: C 67.0; H 8.4; N 25.7%

From the N.M.R. spectroscopy carried out in hexadeuterobenzene it appeared that the product contained 91% of the trans and 9% of the cis isomer.

Example 4

Isolation of trans 2-cyano-3-azabicyclo[3.1.0]hexane

7.0 g of the cis/trans mixture obtained via the procedure described in the previous Example were treated with a cold isopropyl alcohol/petroleum ether mixture from which the trans isomer crystallized in the form of white needles, m.p. 39—40°C.
Yield 6.0 g (85%).

Example 5

Preparation of cis and trans 2-cyano-3-ethoxycarbonyl-3-azabicyclo[3.1.0]hexane

A solution of mixed 2-cyano-3-azabicyclo[3.1.0]hexane (142 g, 1.32 mol. cis/trans ratio 45/55) in water (140 ml) was cooled to 0° and treated with ethyl chloroformate (145 g, 1.33 mol.) over 30 minutes. The temperature was held at 0—2°C by isopropanol/dry ice cooling. A solution of potassium carbonate (181 g, 1.31 mol.) in water (220 ml) was then added dropwise over 30 minutes with the temperature maintained at 0° to −5°C. Reaction was quite exothermic at this stage. The mixture was warmed to 15° over 45 minutes. The layers were separated and the aqueous phase extracted 3 times with diethyl ether (200 ml portions). After washing the combined extracts with water (3 x 200 ml) to pH 7 the ether was removed on a rotary evaporator to give 232 g of crude product.

This material was subjected to vacuum fractionation using a column packed with 15 cm of multi-turn helices. The first fraction obtained was identified using NMR spectroscopy as trans 2-cyano-3-ethoxycarbonyl-3-azabicyclo[3.1.0]hexane. The final fraction obtained was the cis isomer.

Example 6

2-cyano-6-chloro-3-azabicyclo[3.1.0]hexane

Similarly, 2-cyano-6-chloro-3-azabicyclo[3.1.0]hexane was prepared. The single isomer in which the chlorine atom is in the exo position and the cyano group is trans to the CHCl group, was isolated as a yellow liquid which crystallized on standing to give a solid, m.p. 85—87°C.

Analysis:
Calculated: C 50.5; H 5.0; N 19.65
Found: C 50.7; H 5.1; N 19.5

Example 7

Demonstration of herbicidal activity

To evaluate their herbicidal activity the compounds of the invention were tested using as a representative range of plants: maize, *Zea mays* (MZ); barnyard grass, *Echinochloa crusgalli* (BG); oat, *Avena sativa* (O); mustard, *Sinapis alba* (M); sugar beet *Beta vulgaris* (SB); and soya bean, *Glycine maxi* (S).

The tests involved were post-emergence tests, involved foliar spray tests in which seedling plants of the above species were sprayed with a liquid formulation containing a compound of the invention.

The soil used in the tests was a steam-sterilized, modified John Innes Compost mixture in which half the peat, by loose bulk, had been replaced by vermiculite.

The formulations used in the tests were prepared by preparing a solution of the compounds in acetone containing 0.4% by weight of an alkylphenyl/ethylene oxide condensate available under the trade name TRITON X—155. The acetone solution was diluted with an equal volume of water and the resulting formulation applied at a dosage level corresponding to 5 kilograms of active material per hectare in a volume equivalent to 400 litres per hectare.

The herbicidal effects of the compounds were assessed visually seven days after spraying the foliage and eleven days after spraying the soil, and were recorded on a 0—9 scale. A rating 0 indicates no effect on the treated plants, a rating 2 indicates a reduction in growth of the plants of approximately 25%, a rating 5 indicates a reduction of approximately 55%, a rating 9 indicates a reduction of 95%.

The results of the tests are presented in the following Table:

| Compound | Phytotoxicity rating (0–9 scale) | | | | | |
|---|---|---|---|---|---|---|
| | MZ | BG | O | M | SB | S |
| Trans 2-cyano-3-azabicyclo[3.1.0]-hexane | 6 | 5 | 5 | 3 | 3 | 4 |
| 60% cis, 40% trans 2-cyano-3-azabicyclo[3.1.0]-hexane | 2 | 4 | 4 | 2 | 1 | 4 |

## Claims

1. A compound of the general formula:

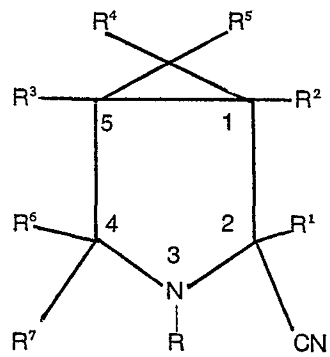

(I)

wherein R represents a hydrogen atom or an alkoxycarbonyl, alkyl or cycloalkyl group which may be unsubstituted or substituted by one or more of the same or different substituents selected from halogen atoms, alkoxy groups and aryl groups; $R^1$ represents a hydrogen atom or an alkyl or cycloalkyl group which may be unsubstituted or substituted by one or more of the same or different substituents selected from halogen atoms and alkoxy groups; and each of $R^2$, $R^3$, $R^4$, $R^5$, $R^6$ and $R^7$ independently represents a hydrogen or halogen atom or an alkyl, cycloalkyl, alkenyl, aryl, alkaryl or aralkyl group which may be unsubstituted or substituted by one or more of the same or different substituents selected from halogen atoms and alkoxy groups, or each of $R^2$, $R^3$, $R^6$ and $R^7$ independently has one of the above meanings and $R^4$ and $R^5$ together represent an alkylene group or each of $R^2$, $R^3$, $R^4$ and $R^5$ has one of the above meanings and $R^6$ and $R^7$ together represent an alkylene group; or an acid addition salt thereof.

2. A compound as claimed in claim 1, in which R represents a hydrogen atom, an alkyl group having up to 6 carbon atoms which may be unsubstituted or substituted by up to 3 halogen atoms, an alkoxycarbonyl group having up to 6 carbon atoms, a cycloalkyl group, or a benzyl group which may be unsubstituted or substituted in the ring by up to 3 halogen atoms.

3. A compound as claimed in either claim 1 or claim 2, in which $R^1$ represents a hydrogen atom or an alkyl group having up to 6 carbon atoms which may be unsubstituted or substituted by up to 3 halogen atoms.

4. A compound as claimed in any one of claims 1 to 3, in which each of $R^2$, $R^3$, $R^4$, $R^5$, $R^6$ and $R^7$ independently represents a hydrogen, fluorine, chlorine or bromine atom or an alkyl or alkenyl group having up to 4 carbon atoms which may be unsubstituted or substituted by up to 3 fluorine, chlorine or bromine atoms, or each of $R^2$, $R^3$, $R^6$ and $R^7$ independently has one of these meanings and $R^4$ and $R^5$ together represent an alkylene group having 4 or 5 carbon atoms, or each of $R^2$, $R^3$, $R^4$ and $R^5$ independently has one of these meanings and $R^6$ and $R^7$ together represent an alkylene group having 4 or 5 carbon atoms.

5. A compound as claimed in claim 1, which is 2-Cyano-3-azabicyclo[3.1.0]hexane or 2-Cyano-3-ethoxycarbonyl-3-azabicyclo[3.1.0]hexane, or an acid addition salt thereof.

6. A process for the preparation of a compound as claimed in any one of claims 1 to 5, characterized in that a compound of the general formula:

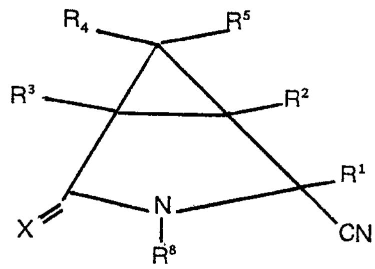

(II)

in which $R^1$, $R^2$, $R^3$, $R^4$ and $R^5$ have the meanings given for the general formula I, X represents an oxygen or sulphur atom, and $R^8$ represents a hydrogen atom or an alkyl, cycloalkyl, aryl, alkaryl or aralkyl group which may be unsubstituted or substituted by one or more of the same or different substituents selected from halogen atoms, alkoxy groups and aryl groups, is reacted with a trialkyl oxoniumfluoroborate, and at least part of the resulting product is reacted with a suitable reducing agent; and if desired, converting the resulting compound of the general formula I into another compound of the general formula I.

7. A process as claimed in claim 6, characterized in that the reaction of the compound of the general formula II with the trialkyloxonium fluoroborate is carried out at a temperature in the range of from 0 to 30°C.

8. A process as claimed in either claim 6 or claim 7, characterized in that the trialkyloxonium fluoroborate is trimethyloxonium fluoroborate or triethyloxonium fluoroborate.

9. A process as claimed in any one of claims 6 to 8, characterized in that the reduction step is carried out using sodium hydride, lithium hydride, lithium borohydride or sodium borohydride.

10. A process as claimed in any one of claims 6 to 9, characterized in that there is prepared a compound of the general formula I in which R represents a hydrogen atom, and that said compound is reacted with a compound of the general formula:

$$Hal - CO_2 - R^9 \qquad (V)$$

in which Hal represents a chlorine or bromine atom and $R^9$ represents an alkyl group which may be unsubstituted or substituted by one or more of the same or different substituents selected from halogen atoms, alkoxy groups and aryl groups, to produce a compound of the general formula I, in which R represents an alkoxycarbonyl group of formula $-CO_2R^9$.

11. A herbicidal composition which comprises a carrier and/or a surface-active agent, characterized in that it contains as active ingredient at least one compound as claimed in any one of claims 1 to 5.

12. A method of controlling undesired plant growth at a locus, characterized in that there is applied to the locus a herbicidally effective amount of a compound as claimed in any one of claims 1 to 5 or a herbicidal composition as claimed in claim 11.

**Revendications**

1. Composé de la formule générale:

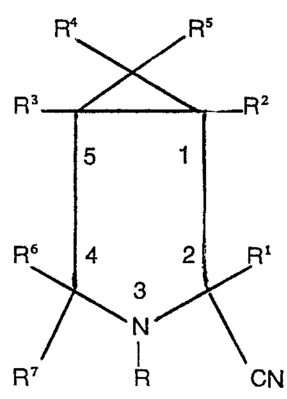

(I)

dans laquelle R représente un atome d'hydrogène ou un groupe alcoxycarbonyle, alcoyle ou cycloalcoyle qui peut être non-substitué ou substitué par un ou plusieurs substituants identiques ou

différents choisis parmi des atomes d'halogènes, des groupes alcoxy et des groupes aryle; $R^1$ représente un atome d'hydrogène ou un groupe alcoyle ou cycloalcoyle qui peut être non-substitué ou substitué par un ou plusieurs substituants identiques ou différents choisis parmi des atomes d'halogènes et des groupes alcoxy; et $R^2$, $R^3$, $R^4$, $R^5$, $R^6$ et $R^7$ représentent chacun indépendamment un atome d'hydrogène ou d'un halogène ou un groupe alcoyle, cycloalcoyle, alcényle, aryle, alcaryle ou aralcoyle qui peut être non-substitué ou substitué par un ou plusieurs substituants identiques ou différents choisis parmi des atomes d'halogènes et des groupes alcoxy, ou $R^2$, $R^3$, $R^6$ et $R^7$ ont chacun indépendamment une des significations ci-dessus et $R^4$ et $R^5$ représentent ensemble un groupe alcoylène ou $R^2$, $R^3$, $R^4$ et $R^5$ ont chacun une des significations ci-dessus et $R^6$ et $R^7$ représentent ensemble un groupe alcoylène; ou un sel d'addition d'acide d'un tel composé.

2. Composé selon la revendication 1, caractérisé en ce que R représente un atome d'hydrogène, un groupe alcoyle ayant jusqu'à 6 atomes de carbone qui peut être non-substitué ou substitué par jusqu'à 3 atomes d'halogènes, un groupe alcoxycarbonyle ayant jusqu'à 6 atomes de carbone, un groupe cycloalcoyle ou un groupe benzyle qui peut être non-substitué ou substitué au noyau par jusqu'à 3 atomes d'halogènes.

3. Composé selon l'une des revendications 1 et 2, caractérisé en ce que $R^1$ représente un atome d'hydrogène ou un groupe alcoyle ayant jusqu'à 6 atomes de carbone qui peut être non-substitué ou substitué par jusqu'à 3 atomes d'halogènes.

4. Composé selon l'une quelconque des revendications 1 à 3 caractérisé en ce que $R^2$, $R^3$, $R^4$, $R^5$, $R^6$ et $R^7$ représentent chacun indépendamment un atome d'hydrogène, de fluor, de chlore ou de brome ou un groupe alcoyle ou alcényle ayant jusqu'à 4 atomes de carbone qui peut être non-substitué ou substitué par jusqu'à 3 atomes de fluor, de chlore ou de brome, ou $R^2$, $R^3$, $R^6$ et $R^7$ ont chacun indépendamment une de ces significations et $R^4$ et $R^5$ représentent ensemble un groupe alcoylène ayant 4 ou 5 atomes de carbone, ou $R^2$, $R^3$, $R^4$ et $R^5$ ont chacun indépendamment une de ces significations et $R^6$ et $R^7$ représentent ensemble un groupe alcoylène ayant 4 ou 5 atomes de carbone.

5. Composé selon la revendication 1, caractérisé en ce que c'est le 2-cyano-3-azabicyclo(3.1.0)hexane ou le 2-cyano-3-éthoxycarbonyl-3-azabicyclo(3.1.0)hexane ou un sel d'addition d'acide de ces composés.

6. Procédé pour la préparation d'un composé tel que défini dans l'une quelconque des revendications 1 à 5, caractérisé en ce qu'un composé de la formule générale:

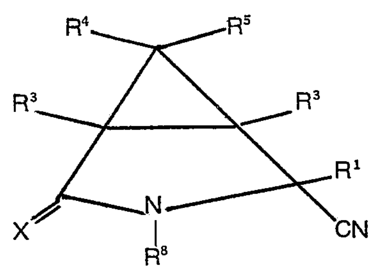

(II)

dans laquelle $R^1$, $R^2$, $R^3$, $R^4$ et $R^5$ ont les significations indiquées pour la formule générale I, X représente un atome d'oxygène ou de soufre et $R^8$ représente un atome d'hydrogène ou un groupe alcoyle, cycloalcoyle, aryle, alcaryle ou aralcoyle qui peut être non-substitué ou substitué par un ou plusieurs substituants identiques ou différents choisis parmi des atomes d'halogènes, des groupes alcoxy et des groupes aryle, est mis à réagir avec un fluoroborate de trialcoyloxonium, et au moins une partie du produit résultant est mise à réagir avec un agent réducteur approprié; et, si on le désire, on transforme le composé résultant de la formule générale I en un autre composé de la formule générale I.

7. Procédé selon la revendication 6, caractérisé en ce que la réaction du composé de la formule générale II avec le fluoroborate de trialcoyloxonium est conduite à une température comprise entre 0 et 30°C.

8. Procédé selon l'une des revendications 6 et 7, caractérisé en ce que le fluoroborate de trialcoyloxonium est du fluoroborate de triméthyloxonium ou du fluoroborate de triéthyloxonium.

9. Procédé selon l'une quelconque des revendications 6 à 8, caractérisé en ce qu'on conduit l'étape de réduction en utilisant de l'hydrure de sodium, de l'hydrure de lithium, du borohydrure de lithium ou du borohydrure de sodium.

10. Procédé selon l'une quelconque des revendications 6 à 9, caractérisé en ce qu'on prépare un composé de la formule générale I dans laquelle R représente un atome d'hydrogène, et que ce composé est mis à réagir avec un composé de la formule générale:

$$Hal - CO_2 - R^9 \quad (V)$$

dans laquelle Hal représente un atome de chlore ou de brome et $R^9$ représente un groupe alcoyle qui peut être non-substitué ou substitué par un ou plusieurs substituants identiques ou différents choisis

parmi des atomes d'halogènes, des groupes alcoxy et des groupes aryle, de manière à produire un composé de la formule générale I, dans laquelle R représente un groupe alcoxycarbonyle de formule $—CO_2R^9$.

11. Composition herbicide qui comprend un véhicule et/ou un agent tensio-actif, caractérisé en ce qu'elle contient comme ingrédient actif au moins un composé tel que revendiqué dans l'une quelconque des revendications 1 à 5.

12. Procédé de lutte contre la croissance de plantes indésirables en un lieu, caractérisé en ce qu'on applique en ce lieu une quantité efficace du point de vue herbicide d'un composé selon l'une quelconque des revendications 1 à 5, ou d'une composition herbicide selon la revendication 11.

**Patentansprüche**

1. Eine Verbindung der allgemeinen Formel:

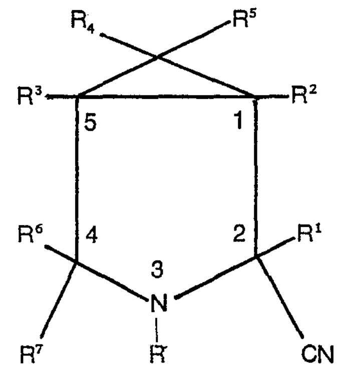

(I)

in der R ein Wasserstoffatom oder eine Alkoxycarbonyl-, Alkyl- oder Cycloalkylgruppe bedeutet, die unsubstituiert oder durch einen oder mehrere gleiche oder verschiedene Substituenten, ausgewählt aus Halogenatomen, Alkoxygruppen und Arylgruppen, substituiert sein kann, $R^1$ ein Wasserstoffatom oder eine Alkyl- oder Cycloalkylgruppe bedeutet, die unsubstituiert oder durch einen oder mehrere gleiche oder verschiedene Substituenten, ausgewählt aus Halogenatomen und Alkoxygruppen, substituiert sein kann, und jedes $R^2$, $R^3$, $R^4$, $R^5$, $R^6$ und $R^7$ unabhängig ein Wasserstoff- oder Halogenatom oder eine Alkyl-, Cycloalkyl-Alkenyl-, Aryl-, Alkaryl- oder Aralkylgruppe bedeutet, die unsubstituiert oder durch einen oder mehrere gleiche oder verschiedene Substituenten, ausgewählt aus Halogenatomen und Alkoxygruppen, substituiert sein kann, oder jedes $R^2$, $R^3$, $R^6$ und $R^7$ unabhängig eine der oben angegebenen Bedeutungen hat und $R^4$ und $R^5$ zusammen eine Alkylengruppe bedeuten oder jedes $R^2$, $R^3$, $R^4$ und $R^5$ eine der oben angegebenen Bedeutungen hat und $R^6$ und $R^7$ zusammen eine Alkylengruppe bedeuten, oder ein Säureadditionssalz dieser Verbindung.

2. Verbindung nach Anspruch 1, in der R ein Wasserstoffatom, eine Alkylgruppe mit bis zu 6 Kohlenstoffatomen, die unsubstituiert oder mit bis zu 3 Halogenatomen substituiert sein kann, eine Alkoxycarbonylgruppe mit bis zu 6 Kohlenstoffatomen, eine Cycloalkylgruppe oder eine Benzylgruppe, die unsubstituiert oder im Ring durch bis zu 3 Halogenatom substituiert sein kann, bedeutet.

3. Verbindung nach Anspruch 1 oder 2, in der $R^1$ ein Wasserstoffatom oder eine Alkylgruppe mit bis zu 6 Kohlenstoffatomen, die unsubstituiert oder durch bis zu 3 Halogenatomen substituiert sein kann, bedeutet.

4. Verbindung nach einem der Ansprüche 1 bis 3, in der jedes $R^2$, $R^3$, $R^4$, $R^5$, $R^6$ und $R^7$ unabhängig ein Wasserstoff-, Fluor-, Chlor- oder Bromatom oder eine Alkyl- oder Alkenylgruppe mit bis zu 4 Kohlenstoffatomen, die unsubstituiert oder durch bis zu 3 Fluor-, Chlor- oder Bromatome substituiert sein kann, bedeutet oder jedes $R^2$, $R^3$, $R^6$ und $R^7$ unabhängig eine dieser Bedeutungen hat und $R^4$ und $R^5$ zusammen eine Alkylengruppe mit 4 oder 5 Kohlenstoffatomen bedeuten oder jedes $R^2$, $R^3$, $R^4$ und $R^5$ unabhängig eine dieser Bedeutungen hat und $R^6$ und $R^7$ zusammen eine Alkylengruppe mit 4 bis 5 Kohlenstoffatomen bedeuten.

5. Verbindung nach Anspruch 1, welche 2-Cyano-3-azabicyclo[3.1.0]hexan oder 2-Cyano-3-äthoxycarbonyl-3-azabicyclo[3.1.0]hexan oder ein Säureadditionssalz davon ist.

6. Verfahren zur Herstellung einer Verbindung nach einem der Ansprüche 1 bis 5, dadurch gekennzeichnet, dass eine Verbindung der allgemeinen Formel:

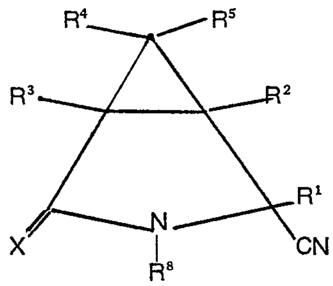

(II)

in der $R^1$, $R^2$, $R^3$, $R^4$ und $R^5$ die für die allgemeine Formel I angegebene Bedeutung haben, X ein Sauerstoffatom oder Schwefelatom bedeutet und $R^8$ ein Wasserstoffatom oder eine Alkyl-, Cycloalkyl-, Aryl-, Alkaryl- oder Aralkylgruppe bedeutet, die unsubstituiert oder durch einen oder mehrere gleiche oder verschiedene Substituenten, ausgewählt aus Halogenatomen, Alkoxygruppen und Arylgruppen, bedeutet, umgesetzt wird mit einem Trialkoxyoniumfluorborat und zumindest ein Teil des entstehenden Produktes umgesetzt wird mit einem geeigneten Reduktionsmittel und, wenn das erwünscht ist, die entstehende Verbindung der allemeinen Formel I in eine andere Verbindung der allgemeinen Formel I umgewandelt wird.

7. Verfahren nach Anspruch 6, dadurch gekennzeichnet, dass die Umsetzung der Verbindung der allgemeinen Formel II mit dem Trialkyloxoniumfluorborat bei einer Temperatur im Bereich von 0 bis 30°C durchgeführt wird.

8. Verfahren nach einem der Ansprüche 6 oder 7, dadurch gekennzeichnet, dass das Trialkyloxoniumfluorborat, Trimethyloxoniumfluorborat oder Triäthyloxoniumfluorborat ist.

9. Verfahren nach einem der Ansprüche 6 bis 8, dadurch gekennzeichnet, dass die Reduktion unter Verwendung von Natriumhydrid, Lithiumhydrid, Lithiumborhydrid oder Natriumborhydrid durchgeführt wird.

10. Verfahren nach einem der Ansprüche 6 bis 9, dadurch gekennzeichnet, dass eine Verbindung der allgemeinen Formel I hergestellt wird, in der R ein Wasserstoffatom bedeutet, und dass diese Verbindung mit einer Verbindung der allgemeinen Formel

$$Hal — CO_2 — R^9 \qquad (V)$$

umgesetzt wird, in der Hal ein Chlor- oder Bromatom bedeutet und $R^9$ eine Alkylgruppe ist, die unsubstituiert oder durch ein oder mehrere gleiche oder verschiedene Substituenten, ausgewählt aus Halogenatom, Alkoxygruppen und Arylgruppen, substituiert ist, um eine Verbindung der allgemeinen Formel I zu erhalten, in der R eine Alkoxycarbonylgruppe der Formel $—CO_2R^9$ bedeutet.

11. Herbizides Mittel umfassend einen Träger und/oder ein oberflächenaktives Mittel, dadurch gekennzeichnet, dass es als Wirkstoff mindestens eine Verbindung nach einem der Ansprüche 1 bis 5 enthält.

12. Verfahren zur Bekämpfung von unerwünschtem Pflanzenwachstum an einer Stelle, dadurch gekennzeichnet, dass auf die Stelle eine herbizid wirksame Menge einer Verbindung nach einem der Ansprüche 1 bis 5 oder eines herbiziden Mittels nach Anspruch 11 aufgebracht wird.